# EUROPEAN PATENT APPLICATION

(11) **EP 1 834 943 A1**
(43) Date of publication of application: **19.09.2007**
(21) Application number: 06713543.4
(22) Date of filing: 06.02.2006
(51) Int. Cl.: C07C 51/08, C07C 65/26, C07B 61/00

(54) **PROCESS FOR PRODUCING AROMATIC DICARBOXYLIC ACID**

(30) Priority: 14.02.2005 JP 2005035530
(71) Applicant: JFE Chemical Corporation, Tokyo 111-0051 (JP)
(72) Inventor: MORI, Hiroaki, e, Taito-ku, Tokyo, 1110051 (JP)
(74) Representative: HOFFMANN EITLE
(86) International application number: PCT/JP2006/302401
(87) International publication number: WO 2006/085630

(57) **Abstract**

The method for manufacturing aromatic dicarboxylic acid according to the formula 1 contains the first step of bringing a nitroaryl nitrile to react with a fluorenylidenebisphenol compound in the presence of an alkaline compound to obtain a corresponding di(cyanoaryloxy) derivative; and the second step of hydrolyzing the di(cyanoaryloxy) derivative to convert into a corresponding dicarboxylic acid, 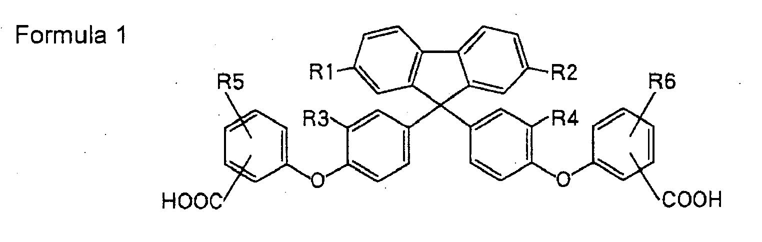 where, R1 to R6 are each independently a hydrogen, an alkyl group, or an aryl group, and they may be the same with or different from each other.

Since the method does not use halide, the load to purification of the product is significantly light, and the aromatic carboxylic acid which is useful as a monomer for manufacturing polymers is readily obtained at a high purity.

## Description

### TECHNICAL FIELD

The present invention relates to a method for manufacturing aromatic dicarboxylic acid.

### BACKGROUND ART

Aromatic dicarboxylic acid derived from a fluorenylidenebisphenol compound is a known compound. The aromatic dicarboxylic acid is a useful compound as a monomer for polymers such as polyester, polyamide, polybenzimidazole, and polybenzoxazole. It is known that these polymers have high heat resistance, mechanical characteristics, resistance to chemicals, and other excellent properties.

In recent years, there have shown rapid developments in the electronics equipment field using these resins, in terms of miniaturization of products, increased packaging density of devices, increased communication speed, and the like. In these current developments of use fields, those resins are requested not only to improve the above characteristics but also to add new characteristics. For polybenzoxazole, as an example, there are requested electric characteristics (low dielectric property and the like), low water-absorption, optical characteristics (transparency, high refractive index, and low birefringence), and the like.

To answer these requirements, there has been developed an aromatic dicarboxylic acid derived from a fluorenylidenebisphenol compound, (hereinafter referred also to as "the aromatic dicarboxylic acid"), as a monomer for above polymers.

For example, the target aromatic dicarboxylic acids are obtained by the method described below. First, the nucleophilic displacement reaction of a dipotassium fluorenylidenebisphenolate compound to 4-fluorobenzonitrile is utilized to synthesize a corresponding di(cyanophenoxy) derivative. Then, two cyano groups in the derivative are subjected to alkaline hydrolysis to convert into a corresponding dicarboxylic acid, (Macromolecular Chemistry and Physics, 200(6), 1428-1433 (1999)). Since, however, the method generates potassium fluoride as a byproduct during the nucleophilic displacement reaction, fluorine corrodes the reactor and significantly affects the environment. In addition, since the fluorine compounds are generally expensive, and since some of fluoroaryl nitriles as the raw material of fluorine compounds are unavailable at an amount of industrial scale, the above exampled method is not practical in view of industrial application.

On the other hand, Japanese Patent Laid-Open No. 62-138449 discloses a method for synthesizing diaryl ether dicarboxylates utilizing the Ullman reaction. For example, diphenyl ether dicarboxylate is synthesized by the reaction between m-hydroxybenzoic acid and p-chlorobenzoic acid in the presence of a copper(I) chloride catalyst and potassium carbonate. The inventor of the present invention tried to synthesize the aromatic dicarboxylic acid derived from a fluorenylidenebisphenol compound using the reaction. However, there occurred an unwelcome side reaction, adding to the target reaction, which side reaction laid load on the purification process, and the yield was not satisfactory.

Furthermore, both the above methods use an aryl halide as the raw material, thereby leaving halide in the final product, though the amount is slight. To this point, not a small number of polymer materials introduced to the electronic materials in recent years are strictly regulated in terms of the residual amount of halides. Accordingly, if the organic halides represented by aryl halide are used for manufacturing aromatic dicarboxylic acids as the monomer, the trace amount of halides in the product has to be removed. The removal of trace amount of halides, however, is extremely difficult.

Therefore, an object of the present invention is to provide an industrially advantageous method for manufacturing aromatic dicarboxylic acid derived from fluorenylidenebisphenol, which aromatic dicarboxylic acid is useful as a monomer for polymer materials.

### DISCLOSURE OF THE INVENTION

That is, the present invention is a method for manufacturing aromatic dicarboxylic acid represented by the formula 1, which method has: the first step of bringing a nitroaryl nitrile to react with a fluorenylidenebisphenol compound in the presence of an alkaline compound to obtain a corresponding di(cyanoaryloxy) derivative; and the second step of hydrolyzing the di(cyanoaryloxy) derivative to convert into a corresponding dicarboxylic acid, 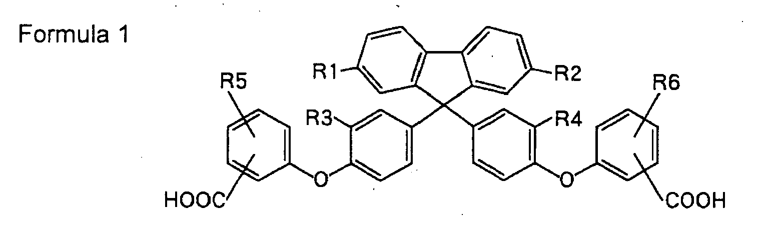 where, R1 to R6 are each independently a hydrogen, an alkyl group, or an aryl group, and they may be the same with or different from each other.

In the manufacturing method, the hydrolysis is preferably the alkaline hydrolysis using a glycol compound as the medium.

The present invention is also a method for manufacturing aromatic dicarboxylic acid represented by the formula 1, which method has: the first step of conducting condensation reaction between a fluorenylidenebisphenol and a nitroaryl nitrile in the presence of an alkaline compound to form an ether linkage, thus obtaining a corresponding dinitrile compound; and the second step of conducting solvolysis of the dinitrile compound in a glycol-based solvent in the presence of an alkaline compound to obtain a corresponding dicarboxylic acid, 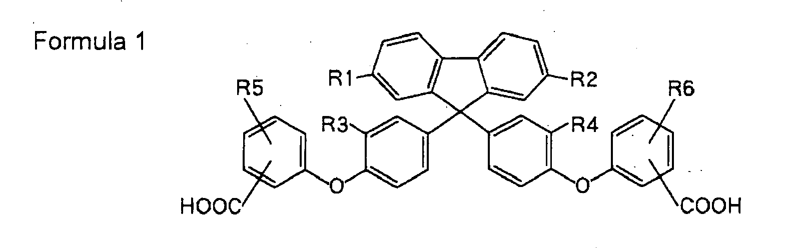 where, R1 to R6 are each independently a hydrogen, an alkyl group, or an aryl group, and they may be the same with or different from each other.

### BEST MODE FOR CARRYING OUT THE INVENTION

The present invention is described below in more detail referring to the preferred embodiments.

The method for manufacturing aromatic dicarboxylic acid according to the present invention has two steps: the first step of bringing a nitroaryl nitrile to conduct condensation reaction with a fluorenylidenebisphenol compound in the presence of an alkaline compound to obtain a corresponding di(cyanoaryloxy) derivative, (hereinafter referred also to as "the dinitrile compound"; and the second step of hydrolyzing the di(cyanoaryloxy) derivative.

The above first step and second step are exemplified by the reaction formulae as below.

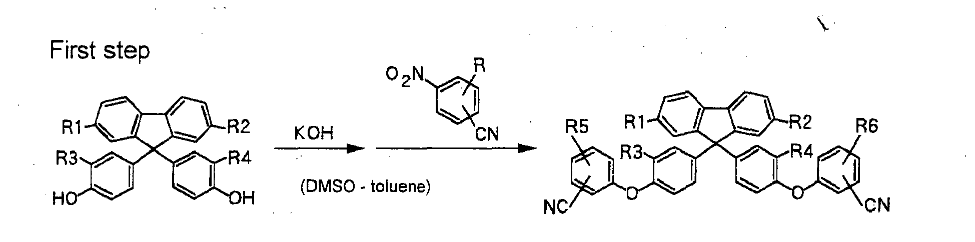

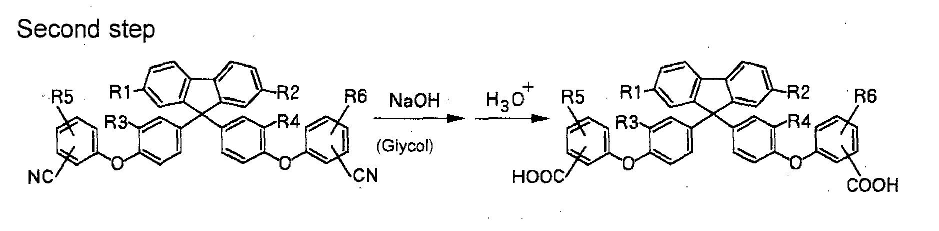

In the first step of the present invention, the nitro group becomes a leaving group to induce the displacement reaction, thus there is no need of using halogen as in the case of related art. As a result, the high purity requested to the monomer for polymers is readily attained.

For the first step, the fluorenylidenebisphenol compound represented by the formula 2 and the nitroaryl nitrile represented by the formula 3, each corresponding to the structure of desired dinitrile compound, may be used.

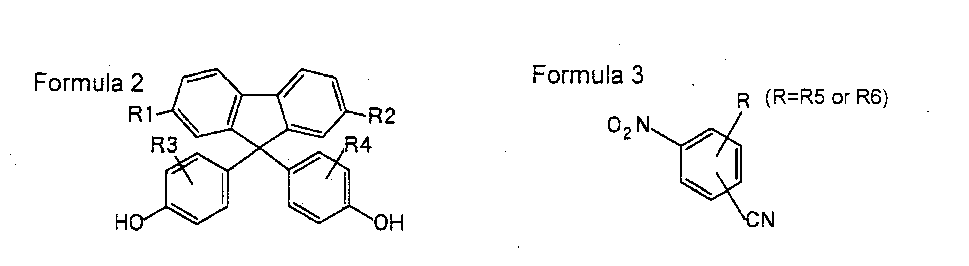

The fluorenylidenebisphenol compound shown in the formula 2 is a compound in which R1 to R4 are each independently a hydrogen, an alkyl group, or an aryl group, and R1 to R4 may be the same with or different from each other. The alkyl group is preferably the one having 1 to 5 carbon atoms, and the aryl group is preferably phenyl group. A specifically preferred substitution position of R3 and R4 is 3-position (or 3'-position) of 4-hydroxyphenyl group in the formula 2. Examples of them are 9,9-bis(4-hydroxyphenyl)fluorene, 9,9-bis(4-hydroxy-3-methylphenyl)fluorene, 9,9-bis(4-hydroxy-3-isopropylphenyl)fluorene, 9,9-bis(4-hydroxy-3-phenylphenyl)fluorene, 9,9-bis(3-allyl-4-hydroxyphenyl)fluorene, 9,9-bis(4-hydroxy-3-naphtylphenyl)fluorene, 2,7-dimethyl-9,9-bis(4-hydroxyphenyl)fluorene, 2,7-dimethyl-9,9-bis(4-hydroxy-3-methylphenyl)fluorene, and 2,7-dimethyl-9,9-bis(4-hydroxy-3-phenylphenyl)fluorene.

Those fluorenylidenebisphenol compounds are easily obtained by a known method in which a corresponding fluorenone or 9, 9-dihalofluorene is brought to dehydration condensation with a corresponding phenol in the presence of an acidic catalyst such as sulfuric acid.

The nitroaryl nitrile shown in the formula 3 is a compound in which R is a hydrogen, an alkyl group, or a phenyl group, and the nitroaryl nitrile may use a combination of different kinds of Rs. The alkyl group is preferably the one having 1 to 5 carbon atoms, and the aryl group is preferably phenyl group. For the aromatic dicarboxylic acid in the formula 1, obtained after the second step, the Rs are each corresponding to R5 and/or R6. Examples of the nitroaryl nitrile are 4-nitrobenzonitrile, 3-nitrobenzonitrile, 2-nitrobenzonitrile, 2-cyano-3-nitrotoluene, 3-cyano-4-nitrotoluene, 4-cyano-3-nitrotoluene, 3-cyano-2-nitrotoluene, 3-cyano-6-nitrotoluene, and 2-cyano-5-nitrotoluene.

The use amount of nitroaryl nitrile to 1 mole of fluorenylidenebisphenol compound is 2 mole or more, preferably 2.2 to 2.5 mole.

The alkaline compound coexisting in the first step is arbitrary one if only it converts the phenolic hydroxyl group in the fluorenylidenebisphenol compound into a metal salt. Examples of that kind of alkaline compound are alkaline metal, alkaline earth metal, or a salt thereof, and preferred ones are at least one compound selected from the group consisting of an alkaline carbonate such as sodium carbonate and potassium carbonate, and an alkaline hydroxide such as sodium hydroxide and potassium hydroxide. As of these, it is specifically preferable to use at least one of alkaline hydroxides. As for the generating metal fluorenylidenebisphenolates, the one prepared separately from the first step can naturally be supplied to the first step.

When an alkaline hydroxide or an alkaline carbonate is brought to react with the fluorenylidenebisphenol compound, there is generated an alkaline metal salt of fluorenylidenebisphenol and water or carbonic acid (water and carbon dioxide gas.). The use amount of alkaline compound in the first step is 2 mole or more, preferably 2.2 to 5 mole, to 1 mole of fluorenylidenebisphenol compound.

A preferred example of the reaction medium used in the first step includes an aprotic polar solvent such as dimethylformamide, dimethylacetamide, dimethylsulfoxide, sulfolane, N-methylpyrrolidone, and 1,3-dimethyl-2-imidazolidinone. These reaction media are preferably used in an amount of 1 to 50 times (by mass) the mass of the fluorenylidenebisphenol compound.

To distilling out the water generated from the reaction between the fluorenylidenebisphenol compound and the alkaline compound to outside the reaction system, a component azeotropic with water preferably coexists with the above aprotic polar solvent. A preferable coexisting component is an aromatic hydrocarbon such as toluene and xylene.

An example of the reaction operation in the first step is described below. A fluorenylidenebisphenol compound, potassium hydroxide, toluene, and dimethylsulfoxide are mixed in a vessel equipped with a stirrer, a thermometer, a Dean-Stalk trap, and a reflux condenser. While keeping nitrogen atmosphere in the system, the contents are heated to about 110°C, and the generating water is distilled out into the Dean-Stalk trap as an azeotrope with toluene. After completing the water distillation, the temperature of the system is lowered to about 100°C to retain the generated dipotassium fluorenylidenebisphenolate compound in the system. A nitroaryl nitrile compound is charged into the vessel to conduct displacement reaction. After continuing the reaction at the temperature (about 100°C) for a specified period, the reaction product mixture is cooled to room temperature. The reaction product mixture is put in a 5% (by weight) sulfuric acid solution, and the deposited crystals are filtered. Thus filtered crude crystals are rinsed with water to remove the residual inorganic salt (alkaline nitrite). The water-rinsed crystals are rinsed by methanol, and then are dried to obtain the target dinitrile compound.

In the succeeding second step, the obtained dinitrile compound is treated by solvolysis, preferably hydrolysis, to convert into dicarboxylic acid or a salt thereof. Solvolysis uses lower alcohol such as methanol, water, and the like. Normally, however, the hydrolysis using water is preferred because of economy. Although the hydrolysis can be done by acid hydrolysis or alkaline hydrolysis, the alkaline hydrolysis is preferred. Specifically preferred one is the alkaline hydrolysis given by dispersing the aromatic dinitrile compound in a glycol medium.

A preferred alkaline compound for the hydrolysis in the second step is at least one compound selected from the group consisting of an alkaline carbonate such as sodium carbonate and potassium carbonate, and an alkaline hydroxide such as sodium hydroxide and potassium hydroxide. As of these, alkaline hydroxide is specifically preferred. The use amount of the alkaline compound is 2 mole or more, preferably 5 to 50 mole, to 1 mole of the dinitrile compound.

The glycol-based medium used in the second step includes ethylene glycol, diethylene glycol, triethylene glycol, glycerin, ethylene glycol monomethylether, diethylene glycol monomethylether, and triethylene glycol monomethylether.

The temperature of hydrolysis is in a range from 20°C to 300°C, preferably from 150°C to 250°C, and the hydrolysis is normally conducted under atmospheric pressure.

After completing the scheduled reactions, the reacted solution is charged into an acidic liquid such as dilute sulfuric acid solution, and the deposited aromatic dicarboxylic acid is collected by filtration. Thus filtered solid is repeatedly rinsed with water, and then is dried by hot air to obtain the crude aromatic dicarboxylic acid. Recrystallization or other treatment is applied to the crude aromatic dicarboxylic acid, at need, to obtain the aromatic dicarboxylic acid of the present invention.

The obtained aromatic dicarboxylic acid represented by the formula 1 contains R1 to R6, each of which is independently a hydrogen, an alkyl group, or an aryl group, and R1 to R6 may be the same with or different from each other. The alkyl group is preferably the one having 1 to 5 carbon atoms, and the aryl group is preferably phenyl group. As of these, specifically preferred one is an aromatic dicarboxylic acid, in which R1 to R6 are each dependently a hydrogen, a methyl group, or a phenyl group, (they may be the same with or different from each other.)

### EXAMPLES

The present invention is described in further detail referring to the examples. The present invention is, however, not limited to these examples. In the following description, "%" is weight basis.

### [Example 1]

To a two-liter four-neck flask equipped with a stirrer, a thermometer, a Dean-Stalk trap, and a reflux condenser, there were poured 74.7 g (0.213 mole) of 9,9-bis(4-hydroxyphenyl)fluorene, 29.9 g (0.533 mole) of potassium hydroxide, 50 g of toluene, and 790 g of dimethylformamide. While agitating the contents and keeping the system in a nitrogen atmosphere, the system was heated to 110°C, thus the generated water was distilled out as an azeotrope with toluene. After completing the water distillation, the temperature of the system was lowered to 100°C. A 79.0 g (0.533 mole) of 4-nitrobenzonitrile was charged into the flask to execute reaction at the temperature for 3 hours. After completing the reaction, the reaction product mixture was cooled to room temperature, which was then charged into a 1500 g of 5% hydrochloric acid solution. The deposited crystals were collected by filtration. Thus collected crude crystals were rinsed with 1000 g of water, followed by filtering to remove the residual inorganic salt. The crystals were again rinsed with water and filtered. The crystals were then rinsed with 500 g of methanol and filtered. After repeated the methanol-rinsing and drying once more, the crystals were dried to obtain 104 g of dinitrile compound. The yield of the dinitrile compound was 88.3% on the basis of 9,9-bis(4-hydroxyphenyl)fluorene.

Then, to a two-liter four-neck flask equipped with a stirrer, a thermometer, and a reflux condenser, there were poured 104 g (equivalent to 0.186 mole) of the dinitrile compound, 104 g of potassium hydroxide, and 832 g of ethylene glycol. The mixture was brought to react at 160°C for 2 hours. After completing the reaction, the reaction product mixture was cooled, and was charged into 3 liter of 10% sulfuric acid solution. The mixture was agitated at room temperature for about 30 min to 1 hour. Then, the free aromatic dicarboxylic acid was separated by filtration. The aromatic dicarboxylic acid was dispersed in 1.5 liter of 10% sulfuric acid solution to execute heat treatment at 80°C. After filtering the mixture to separate the solid aromatic dicarboxylic acid, two times of rinsing with 1. 5 liter of boiled distilled water were applied. The obtained aromatic dicarboxylic acid was dried by hot air, and was recrystallized using 400 g of dioxane to obtain 100 g of 9,9-bis[4-(4-carboxyphenoxy)phenyl]fluorene. The yield of the product was 90.0% on the basis of dinitrile compound, and the purity of the product determined by high performance liquid chromatography was 99.5%.

Thus obtained aromatic dicarboxylic acid showed characteristic absorption position of substituent according to infrared absorption spectra and element analysis as follows.
Infrared absorption spectra
Carbonyl group: 1703 cm⁻¹
Hydroxyl group: 2600-3400 cm⁻¹
Ether group: 1238 cm⁻¹
Element analysis (Molecular formula: C₃₉H₂₆O₆)

**Table 1**

| | C (%) | H (%) | O (%) |
|---|---|---|---|
| Observed value | 79.22 | 4.47 | 16.31 |
| Calculated value | 79.31 | 4.44 | 16.25 |

### [Example 2]

The same procedure to that of Example 1 was applied except that the nitroaromatic nitrile was changed from 4-nitrobenzonitrile to 2-nitrobenzonitrile, thus obtained 91 g of 9,9-bis[4-(2-carboxyphenoxy)phenyl]fluorene. The yield of the product was 72.3% on the basis of 9, 9-bis (4-hydroxyphenyl) fluorene, and the purity of the product determined by high performance liquid chromatography was 99.3%.

Thus obtained aromatic dicarboxylic acid showed characteristic absorption position of substituent according to infrared absorption spectra and element analysis as follows.
Infrared absorption spectra
Carbonyl group: 1709 cm⁻¹
Hydroxyl group: 2600-3400 cm⁻¹
Ether group: 1231 cm⁻¹
Element analysis (Molecular formula: C₃₉H₂₆O₆)

**Table 2**

| | C (%) | H (%) | O (%) |
|---|---|---|---|
| Observed value | 79.26 | 4.48 | 16.26 |
| Calculated value | 79.31 | 4.44 | 16.25 |

### [Example 3]

The same procedure to that of Example 1 was applied except that the fluorenylidenebisphenol compound was changed from 9,9-bis(4-hydroxyphenyl)fluorene to 80.6 g (0.213 mole) of 9,9-bis(4-hydroxy-3-methylphenyl)fluorene, thus obtained 100 g of 9,9-bis[4-(4-carboxyphenoxy)-3-methylphenyl]fluorene. The yield of the product was 75.9% on the basis of 9,9-bis(4-hydroxy-3-methylphenyl)fluorene, and the purity of the product determined by high performance liquid chromatography was 99.6%.

Thus obtained aromatic dicarboxylic acid showed characteristic absorption position of substituent according to infrared absorption spectra and element analysis as follows.
Infrared absorption spectra
Carbonyl group: 1701 cm⁻¹
Hydroxyl group: 2600-3400 cm⁻¹
Ether group: 1226 cm⁻¹
Element analysis (Molecular formula: C₄₁H₃₀O₅)

**Table 3**

| | C (%) | H (%) | O (%) |
|---|---|---|---|
| Observed value | 79.50 | 4.93 | 15.57 |
| Calculated value | 79.59 | 4.89 | 15.52 |

### [Example 4]

The same procedure to that of Example 1 was applied except that the fluorenylidenebisphenol compound was changed from 9,9-bis(4-hydroxyphenyl)fluorene to 106.9 g (0.213 mole) of 9,9-bis(4-hydroxy-3-phenylphenyl)fluorene, thus obtained 113 g of 9,9-bis[4-(4-carboxyphenoxy)-3-phenylphenyl]fluorene. The yield of the product was 71.4% on the basis of 9,9-bis(4-hydroxy-3-phenylphenyl)fluorene, and the purity of the product determined by high performance liquid chromatography was 98.7%.

Thus obtained aromatic dicarboxylic acid showed characteristic absorption position of substituent according to infrared absorption spectra and element analysis as follows.
Infrared absorption spectra
Carbonyl group: 1698 cm⁻¹
Hydroxyl group: 2600-3400 cm⁻¹
Ether group: 1221 cm⁻¹
Element analysis (Molecular formula: C₅₁H₃₄O₆)

**Table 4**

| | C (%) | H (%) | O (%) |
|---|---|---|---|
| Observed value | 82.36 | 4.60 | 13.04 |
| Calculated value | 82.46 | 4.62 | 12.92 |

As described above, the method according to the present invention allows manufacturing an aromatic dicarboxylic acid having a fluorene skeleton at a high yield, which aromatic dicarboxylic acid is useful as a raw material of high functional polymers. Since the method does not use halide, the load to the product purification is significantly light, and the high purity aromatic carboxylic acid is easily obtained.

### INDUSTRIAL APPLICABILITY

The method of the present invention generates an aromatic dicarboxylic acid having a fluorene skeleton at a high yield and high purity with a light purification load. The aromatic dicarboxylic acid is a useful compound as a monomer of high functional polymers such as polyester, polyamide, polybenzimidazole, and polybenzoxazole, and is able to supply to a wide range of industries.

## Claims

1. A method for manufacturing aromatic dicarboxylic acid represented by the formula 1, comprising: a first step of conducting reaction between a fluorenylidenebisphenol compound and a nitroaryl nitrile in the presence of an alkaline compound to obtain a corresponding di(cyanoaryloxy) derivative; and a second step of conducting hydrolysis of the di(cyanoaryloxy) derivative to convert into a corresponding dicarboxylic acid, 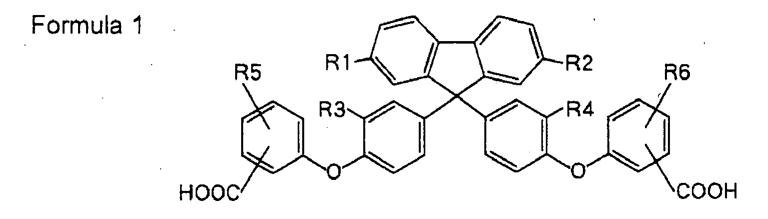 where, R1 to R6 are each independently a hydrogen, an alkyl group, or an aryl group, and they may be the same with or different from each other.

2. The method for manufacturing aromatic dicarboxylic acid according to claim 1, wherein the hydrolysis is alkaline hydrolysis using a glycol compound as the medium.

3. A method for manufacturing aromatic dicarboxylic acid represented by the formula 1, comprising: a first step of conducting condensation reaction between a fluorenylidenebisphenol and a nitroaryl nitrile in the presence of an alkaline compound to form an ether linkage, thus obtaining a corresponding dinitrile compound; and a second step of conducting solvolysis of the dinitrile compound in a glycol solvent in the presence of an alkaline compound to obtain a corresponding dicarboxylic acid, 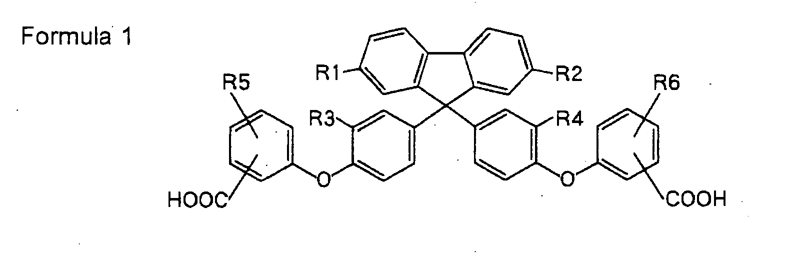 where, R1 to R6 are each independently a hydrogen, an alkyl group, or an aryl group, and they may be the same with or different from each other.
